# EUROPÄISCHE PATENTSCHRIFT

(11) **EP 1 967 213 B1**
(45) Veröffentlichungstag und Bekanntmachung des Hinweises auf die Patenterteilung: **26.02.2014**
(21) Anmeldenummer: 08000800.6
(22) Anmeldetag: 17.01.2008
(51) Int. Cl.: A61L 15/46, A61L 2/08

(54) **Verfahren zur Herstellung eines Verbandstoffes**
Method for manufacturing a dressing
Procédé de fabrication d'un pansement

(30) Priorität: 05.03.2007 EP 07004439
(43) Veröffentlichungstag der Anmeldung: 10.09.2008
(73) Patentinhaber: NOBA Verbandmittel Danz GmbH u. Co. KG, 58300 Wetter-Wengern (DE)
(72) Erfinder: Danz, Anja Annemarie Dr., 58300 Wetter (DE); Danz, Sebastian, 58300 Wetter (DE)
(74) Vertreter: Rohmann, Michael

(56) Entgegenhaltungen:
- EP-A2- 0 197 217
- WO-A-01/70577
- WO-A-99/42142
- GB-A- 1 241 887
- GB-A- 189 718 178

## Beschreibung

Die Erfindung betrifft ein Verfahren zur Herstellung eines Verbandstoffes, wobei ein Trägermaterial aus natürlichen Fasern und/oder aus Kunststofffasern mit zumindest einem Wirkstoff beschichtet bzw. imprägniert wird, wobei als Wirkstoff Jodoform eingesetzt wird. Bei dem beschichteten Verbandstoff kann es sich insbesondere um eine Mull- oder Tamponadebinde handeln. Grundsätzlich können im Rahmen der Erfindung aber auch andere Verbandstoffe hergestellt werden.

Aus der Praxis, beispielsweise aus GB18178A, ist es grundsätzlich bekannt, zur Herstellung eines Verbandstoffes einen Wirkstoff auf ein Trägermaterial aufzubringen, wobei es sich bei dem Wirkstoff insbesondere um ein Antiseptikum handeln kann. Als Antiseptikum wird beispielsweise Jodoform eingesetzt. Diese bekannten Verbandstoffe werden insbesondere auf Wunden aufgebracht und der Wirkstoff wird bei Kontakt mit der Wunde nach und nach freigesetzt. Der Erfindung liegt jedoch die Erkenntnis zugrunde, dass trotz der Beschichtung des Trägermaterials mit dem Wirkstoff bzw. mit dem Antiseptikum nichtsdestoweniger in unerwünschter Weise Keime in die Wunde gelangen können.

Dementsprechend liegt der Erfindung das technische Problem zugrunde, ein Verfahren der eingangs genannten Art anzugeben, mit dem keimfreie Verbandstoffe hergestellt werden können und insbesondere Verbandstoffe, aus denen bei einem Erstkontakt mit einer Wunde keine Keime in die Wunde gelangen können. Der Erfindung liegt weiterhin das technische Problem zugrunde, einen entsprechenden Verbandstoff anzugeben.

Zur Lösung dieses technischen Problems lehrt die Erfindung ein Verfahren zur Herstellung eines beschichteten sterilen Verbandstoffes, wobei ein Trägermaterial aus natürlichen Fasern und/oder aus Kunststofffasern mit zumindest einem Wirkstoff beschichtet bzw. imprägniert wird, wobei als Wirkstoff Jodoform eingesetzt wird
und wobei das beschichtete Trägermaterial im Anschluss daran mit Elektronenstrahlung sterilisiert wird. Die Sterilisierung des beschichteten Trägermaterials erfolgt also durch Bestrahlung mit Hilfe von Elektronenstrahlung.

Bei dem Trägermaterial bzw. bei dem Verbandstoff handelt es sich vorzugsweise um eine Mull- oder Tamponadebinde. Das Trägermaterial kann gemäß einer bevorzugten Ausführungsform aus natürlichen Fasern bestehen, insbesondere aus Baumwollfasern. Nach einer anderen bevorzugten Ausführungsform kann das Trägermaterial aber auch aus Kunststofffasern bestehen, insbesondere aus Viskosefasern. Es liegt im Rahmen der Erfindung, dass es sich bei dem Trägermaterial um ein gewebtes Trägermaterial handelt. Grundsätzlich kann als Trägermaterial aber auch ein nicht gewebtes Trägermaterial, insbesondere ein Nonwoven-Vlies verwendet werden. Zweckmäßigerweise wird ein saugfähiges Trägermaterial bzw. ein saugfähiges Gewebe als Trägermaterial eingesetzt. Das Trägermaterial wird also mit dem Wirkstoff Jodoform (Trijodmethan) als einem Antiseptikum beschichtet bzw. imprägniert.

Es liegt im Rahmen der Erfindung, dass als Wirkstoff zumindest ein Antiseptikum und/oder zumindest ein Haemostyptikum und/oder zumindest ein Desinfiziens und/oder zumindest eine wundheilungsfördernde Substanz eingesetzt wird. Der Wirkstoff kann auch unter mehrere der vorgenannten Oberbegriffe fallen. Besonders bewährt hat sich im Rahmen des erfindungsgemäßen Verfahrens der Einsatz zumindest eines Antiseptikums als Wirkstoff.

Eine empfohlene Ausführungsform der Erfindung ist dadurch gekennzeichnet, dass eine Lösung des Wirkstoffes auf das Trägermaterial aufgebracht wird. Es liegt dabei im Rahmen der Erfindung, dass die Lösung den festen Wirkstoff, vorzugsweise Jodoform in gelöster Form enthält. Zweckmäßigerweise wird der feste Wirkstoff - insbesondere Jodoform - in einem flüssigen Lösungsmittel gelöst. Nach bevorzugter Ausführungsform wird der feste Wirkstoff - insbesondere Jodoform - in einem Lösungsmittel aus der Gruppe "Ethanol, Aceton, Chloroform, Glycerol, Ether" gelöst. Das Trägermaterial wird dann mit der Wirkstofflösung - insbesondere Jodoformlösung - imprägniert bzw. getränkt. Hierzu wird empfohlenermaßen streifenförmiges Trägermaterial durch die Wirkstofflösung hindurchgeführt. Grundsätzlich könnte das Trägermaterial auch mit der Wirkstofflösung besprüht werden.

Eine besonders bevorzugte Ausführungsform der Erfindung ist dadurch gekennzeichnet, dass zumindest eine die Haftung des Wirkstoffes fördernde Substanz auf das Trägermaterial aufgebracht wird. Diese Substanz fördert bzw. erhöht also die Haftung des Wirkstoffes an dem Trägermaterial. Es empfiehlt sich, dass die zumindest eine die Haftung des Wirkstoffes fördernde Substanz in der vorzugsweise eingesetzten Wirkstofflösung enthalten ist. Zweckmäßigerweise wird zumindest ein Paraffin als die Haftung des Wirkstoffes fördernde Substanz auf das Trägermaterial aufgebracht. Bevorzugt handelt es sich dabei um ein flüssiges bzw. dickflüssiges Paraffin oder um Glycerol, empfohlenermaßen 85%-iger Glycerol.

Zum Aufbringen des Wirkstoffes auf das Trägermaterial wird nach bevorzugter Ausgestaltung der Erfindung eine Wirkstofflösung eingesetzt, die den Wirkstoff - insbesondere Jodoform - in einem Lösungsmittel gelöst enthält und vorzugsweise zusätzlich die haftungsfördernde Substanz - insbesondere zumindest ein Paraffin oder Glycerol - enthält.

Es liegt im Rahmen der Erfindung, dass das Trägermaterial nach dem Aufbringen des Wirkstoffes bzw. der Wirkstofflösung getrocknet wird. Nach empfohlener Ausführungsform erfolgt die Trocknung des Trägermaterials bei einer Temperatur von 25° C bis 70° C, bevorzugt 30° C bis 60° C und sehr bevorzugt 30° C bis 50° C.

Eine sehr bevorzugte Ausführungsform der Erfindung ist dadurch gekennzeichnet, dass das mit dem Wirkstoff beschichtete bzw. imprägnierte Trägermaterial zuerst verpackt und danach mit Elektronenstrahlung bzw. β-Strahlung sterilisiert wird. Nach einer Ausführungsvariante wird zur Verpackung zunächst das streifenförmige imprägnierte Trägermaterial aufgewickelt. Der Wickel des imprägnierten Trägermaterials wird dann zweckmäßigerweise in eine Primärverpackung eingesetzt, die aus einem licht- und luftundurchlässigen Material besteht, insbesondere aus Kunststoff oder Aluminiumverbundmaterial. Diese Primärverpackung kann in eine Sekundärverpackung eingesetzt werden. Es liegt im Rahmen der Erfindung, dass die Primärverpackung in eine Faltschachtel, insbesondere aus Pappe überführt wird. Vorzugsweise werden mehrere Faltschachteln in einem Karton untergebracht. Dann ist ein bevorzugter endverpackter Zustand des imprägnierten Trägermaterials in einer Tertiärverpackung erreicht.

Nach einer bevorzugten Ausführungsform der Erfindung wird das endverpackte imprägnierte Trägermaterial mit der Elektronenstrahlung bzw. mit der β- Strahlung sterilisiert. Endverpackung bzw. endverpackter Zustand meint insbesondere, dass der imprägnierte Verbandstoff in einer Primärverpackung untergebracht ist und dass sich diese Primärverpackung in zumindest einer weiteren Verpackung befindet. Die Elektronenbestrahlung bzw. die Bestrahlung mit β -Strahlen erfolgt vorzugsweise von gegenüberliegenden Seiten der Verpackung bzw. der Endverpackung.

Gemäß einer besonders empfohlenen Ausführungsform der Erfindung wird die Elektronenstrahlsterilisation bzw. die β-Strahlungssterilisation mit einer Dosis von 10 bis 75 kGy (10.000-75.000J/kg), insbesondere von 15 bis 65 kGy (15.000-65.000J/kg), bevorzugt von 20 bis 60 kGy (20.000-60.000J/kg) und sehr bevorzugt von 25 bis 55 kGy (25.000-55.000J/kg) durchgeführt.

Der Erfindung liegt die Erkenntnis zugrunde, dass mit dem erfindungsgemäßen Verfahren auf einfache und effektive Weise ein steriler Verbandstoff hergestellt werden kann. Der Erfindung liegt insbesondere die Erkenntnis zugrunde, dass das Trägermaterial zunächst mit dem Wirkstoff, vorzugsweise mit einem Antiseptikum beschichtet werden muss und anschließend erfindungsgemäß mit Elektronenstrahlung bzw. β-Strahlung sterilisiert werden muss. Ein auf diese erfindungsgemäße Weise behandeltes Trägermaterial liefert den sterilen Verbandstoff. Beim Aufbringen dieses Verbandstoffes auf eine Wunde können also keine Keime mehr in die Wunde gelangen obwohl die Wirkung des Wirkstoffes erst nach einiger Zeit einsetzt. Im Rahmen der Erfindung kommt es insbesondere auf die Strahlungsart, nämlich die Elektronenstrahlung an. Der Erfindung liegt außerdem die Erkenntnis zugrunde, dass andere Arten von Sterilisationsbestrahlungen, insbesondere andere Arten von radioaktiven Bestrahlungen bzw. elektromagnetischen Bestrahlungen nicht zu dem erfindungsgemäßen Erfolg führen, da sich dabei in der Regel der Wirkstoff zersetzt.

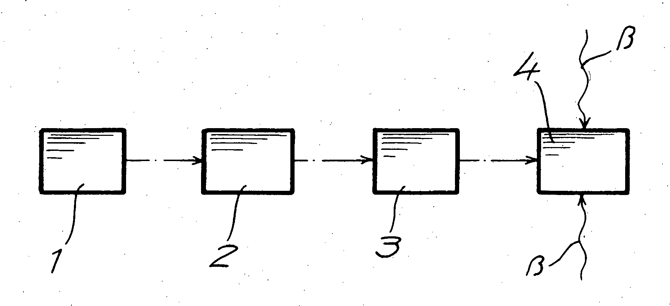

Nachfolgend wird die Erfindung anhand einer ein Ausführungsbeispiel darstellenden Zeichnung näher erläutert. Die einzige Figur zeigt ein Verfahrensschema für das erfindungsgemäße Verfahren. Ein Trägermaterial, beispielsweise in Form eines saugfähigen Gewebes aus natürlichen Fasern, insbesondere Baumwollfasern, wird in der Beschichtungs- bzw. Imprägnierungsstation 1 zunächst mit einer Jodoformlösung imprägniert. Dazu wird das streifenförmige Trägermaterial zweckmäßigerweise durch die Jodoformlösung geführt. Im Anschluss daran erfolgt eine Trocknung des beschichteten/ imprägnierten Trägermaterials in der Trocknungsstation 2. Daraufhin wird das getrocknete imprägnierte Trägermaterial in der Verpackungsstation 3 verpackt und endverpackt. In der Bestrahlungsstation 4 wird dann vorzugsweise das endverpackte Produkt von zwei gegenüberliegenden Seiten mit β -Strahlung bestrahlt. Das ist in der Figur angedeutet. Der fertige Verbandstoff ist nach dieser erfindungsgemäßen Behandlung steril.

## Patentansprüche

1. Verfahren zur Herstellung eines Verbandstoffes, wobei ein Trägermaterial aus natürlichen Fasern und/oder aus Kunststofffasern mit zumindest einem Wirkstoff beschichtet bzw. imprägniert wird,
wobei als Wirkstoff Jodoform eingesetzt wird,
und wobei das beschichtete Trägermaterial im Anschluss daran mit Elektronenstrahlung sterilisiert wird.

2. Verfahren nach Anspruch 1, wobei als Wirkstoff zumindest ein Antiseptikum und/oder zumindest ein Haemostyptikum und/oder zumindest ein Desinfiziens und/oder zumindest eine wundheilungsfördernde Substanz eingesetzt wird.

3. Verfahren nach einem der Ansprüche 1 oder 2, wobei eine Lösung des Wirkstoffes auf das Trägermaterial aufgebracht wird.

4. Verfahren nach einem der Ansprüche 1 bis 3, wobei zumindest eine die Haftung des Wirkstoffes fördernde Substanz auf das Trägermaterial aufgebracht wird.

5. Verfahren nach einem der Ansprüche 1 bis 4, wobei das mit dem Wirkstoff beschichtete bzw. imprägnierte Trägermaterial zuerst verpackt und danach mit Elektronenstrahlung sterilisiert wird.

6. Verfahren nach einem der Ansprüche 1 bis 5, wobei die Elektronenstrahlsterilisation mit einer Dosis von 10.000 bis 75.000 J/kg (10 bis 75 kGy), vorzugsweise von 15.000 bis 65.000 J/kg (15 bis 65 kGy), bevorzugt von 20.000 bis 60.000 J/kg (20 bis 60 kGy) und sehr bevorzugt von 25.000 bis 55.000 J/kg (25 bis 55 kGy) durchgeführt wird.

## Claims

1. A method for producing a dressing, wherein a substrate material made of natural fibres and/or of plastic fibres is coated or impregnated with at least one active ingredient,
wherein triiodomethane is used as active ingredient,
and wherein the coated substrate material is then sterilised with electron radiation.

2. The method according to Claim 1, wherein at least one antiseptic and/or at least one haemostyptic and/or at least one disinfectant and/or at least one wound-healing-promoting substance is/are used as active ingredient.

3. The method according to one of Claims 1 or 2, wherein a solution of the active ingredient is applied to the substrate material.

4. The method according to one of Claims 1 to 3, wherein at least one substance promoting the adhesion of the active ingredient is applied to the substrate material.

5. The method according to one of Claims 1 to 4, wherein the substrate material coated or impregnated with the active ingredient is first packaged and then sterilised with electron radiation.

6. The method according to one of Claims 1 to 5, wherein the electron beam sterilisation is performed with a dose from 10,000 to 75,000 J/kg (10 to 75 kGy), preferably from 15,000 to 65,000 J/kg (15 to 65 kGy), preferably from 20,000 to 60,000 J/kg (20 to 60 kGy), and very preferably from 25,000 to 55,000 J/kg (25 to 55 kGy).

## Revendications

1. Procédé de fabrication d'une gaze, dans lequel un matériau porteur en fibres naturelles et/ou en fibres synthétiques est revêtu, respectivement, imprégné d'au moins une substance active,
sachant que du iodoforme est employé comme substance active,
et sachant que le matériau porteur revêtu est stérilisé ensuite par faisceau d'électrons.

2. Procédé selon la revendication 1, dans lequel on emploie en tant que substance active, au moins un antiseptique et/ou au moins un hémostyptique et/ou au moins un désinfectant et/ou au moins une substance favorisant la guérison de la plaie.

3. Procédé selon la revendication 1 ou 2, dans lequel une solution de la substance active est appliquée sur le matériau porteur.

4. Procédé selon l'une des revendications 1 à 3, dans lequel au moins une substance favorisant l'adhérence de la substance active est appliquée sur le matériau porteur.

5. Procédé selon l'une des revendications 1 à 4, dans lequel le matériau porteur revêtu, respectivement, imprégné, est d'abord emballé puis stérilisé par faisceau d'électrons.

6. Procédé selon l'une des revendications 1 à 5, dans lequel la stérilisation par faisceau d'électrons est effectuée avec une dose de 10 000 à 75 000 J/kg (10 à 75 kGy), de préférence de 15 000 à 65 000 J/kg (15 à 65 kGy), de préférence de 20 000 à 60 000 J/kg (20 à 60 kGy) et de manière vraiment préférée de 25 000 à 55 000 J/kg (25 à 55 kGy).
